# EUROPEAN PATENT APPLICATION

(11) **EP 4 579 005 A1**
(43) Date of publication of application: **02.07.2025**
(21) Application number: 24210035.2
(22) Date of filing: 31.10.2024
(51) Int. Cl.: C30B 23/02, C30B 29/36

(54) **SIC INGOT AND METHOD FOR MANUFACTURING SIC SUBSTRATE**

(30) Priority: 28.12.2023 JP 2023223189; 28.12.2023 JP 2023223451; 28.12.2023 JP 2023223461; 25.10.2024 JP 2024188125; 25.10.2024 JP 2024188186; 25.10.2024 JP 2024188277
(71) Applicant: Resonac Corporation, Tokyo 105-7325 (JP)
(72) Inventor: Noguchi, Shunsuke, Tokyo, 105-7325 (JP)
(74) Representative: Strehl & Partner mbB

(57) **Abstract**

This SiC ingot includes a step-flow growth region and a facet, in a cut surface that passes through a center and is in a <11-20> direction, an inner boundary between the facet and the step-flow growth region has a first inclined surface inclined in a [-1-120] direction with respect to a crystal growth direction and a second inclined surface inclined in a [11-20] direction with respect to the crystal growth direction, and an inflection point between the first inclined surface and the second inclined surface is located on a side of a first end, which is a Si plane or a plane inclined by an offset angle from the Si plane, from a center position of an ingot length.

## Description

### BACKGROUND OF THE INVENTION

### Field of the Invention

The present invention relates to a SiC ingot and a method for manufacturing a SiC substrate.

The present application claims priority on Japanese Patent Application No. 2023-223189 filed on December 28, 2023, Japanese Patent Application No. 2023-223451 filed on December 28, 2023, Japanese Patent Application No. 2023-223461 filed on December 28, 2023, Japanese Patent Application No. 2024-188125 filed on October 25, 2024, Japanese Patent Application No. 2024-188186 filed on October 25, 2024, Japanese Patent Application No. 2024-188277 filed on October 25, 2024, the content of which is incorporated herein by reference.

### Description of Related Art

Silicon carbide (SiC) has a dielectric breakdown field one order of magnitude larger than silicon (Si) and a band gap three times larger than silicon (Si). In addition, silicon carbide (SiC) has properties such as a thermal conductivity about three times higher than silicon (Si). For this reason, silicon carbide (SiC) is expected to be applied to power devices, high frequency devices, high temperature operating devices, and the like. For this reason, in recent years, SiC epitaxial wafers have come to be used for the above-described semiconductor devices.

A SiC epitaxial wafer is obtained by stacking a SiC epitaxial layer on the surface of a SiC substrate. Hereinafter, the substrate before the SiC epitaxial layer is stacked thereon is referred to as a SiC substrate, and the substrate after the SiC epitaxial layer is stacked thereon is referred to as a SiC epitaxial wafer. The SiC substrate is cut out from a SiC ingot. A SiC ingot is obtained by performing crystal growth of a SiC single crystal on a seed crystal.

For example, as described in Patent Document 1, when a SiC ingot is produced, facets are formed in the SiC ingot. During the crystal growth of the SiC ingot, a part of a crystal growth surface becomes parallel to a c plane, and a surface parallel to the c plane is exposed on the crystal growth surface. This surface parallel to the c plane has a different pattern in crystal growth from other crystal growth surfaces on which step-flow growth is performed. A portion obtained through the crystal growth in a different pattern from a portion obtained through the step-flow growth is a facet.

### [Patent Documents]

[Patent Document 1] Japanese Patent No. 6050053

### SUMMARY OF THE INVENTION

The facet has a lower resistance value than the portion obtained through the step-flow growth. This is because the amount of nitrogen taken in is relatively increased on the facet. A difference in resistivity between the facet and a step-flow growth region adversely affects laser processing and electric discharge processing. For example, in a case in which cracks are created in a SiC ingot with a laser and a SiC substrate is cut out from the SiC ingot, it is necessary to change a laser output at a boundary between the facet and the step-flow growth region. The greater the number of times that the laser output is changed, the lower the throughput of the processing. On the other hand, if the facet becomes too small, especially in the early stages of growth, threading screw dislocations (TSDs) cannot be sufficiently introduced into the facet, and thus heterogeneous polymorphs are likely to occur.

The present invention has been made in consideration of the above-described problems, and an object of the present invention is to provide a SiC ingot for achieving both crystal quality and easiness of processing and a method for manufacturing a SiC substrate using the SiC ingot.

The present invention provides the following solutions to solve the above problems.
(1) A SiC ingot according to a first aspect includes a step-flow growth region and a facet. In the SiC ingot, in a cut surface that passes through a center and is in a <11-20> direction, an inner boundary between the facet and the step-flow growth region has a first inclined surface inclined in a [-1-120] direction with respect to a crystal growth direction and a second inclined surface inclined in a [11-20] direction with respect to the crystal growth direction. An inflection point between the first inclined surface and the second inclined surface is located on a side of a first end, which is a Si plane or a plane inclined by an offset angle from the Si plane, from a center position of an ingot length.
(2) In the SiC ingot according to the above aspect (1), the inflection point between the first inclined surface and the second inclined surface may be located on a side of the first end side from a position shifted by 40% of the ingot length in the crystal growth direction from the first end.
(3) In the SiC ingot according to the above aspect (1) or (2), the first inclined surface may be closer to the first end than the second inclined surface.
(4) In the SiC ingot according to any one of the above aspects (1) to (3), an inclination angle of the first inclined surface with respect to the crystal growth direction may be smaller than an inclination angle of the second inclined surface with respect to the crystal growth direction.
(5) In the SiC ingot according to any one of the above aspects (1) to (4), the first inclined surface and the second inclined surface may include a portion in which an absolute value of an inclination angle with respect to the crystal growth direction is 5° or more.
(6) In the SiC ingot according to any one of the above aspects (1) to (5), at least one of the first inclined surface and the second inclined surface may include a portion in which an absolute value of an inclination angle with respect to the crystal growth direction is 15° or more.
(7) In the SiC ingot according to any one of the above aspects (1) to (6), the facet may be located in a [11-20] direction from a plane that passes through a center in a <11-20> direction and is orthogonal to the <11-20> direction.
(8) In the SiC ingot according to any one of the above aspects (1) to (7), the ingot length may be 10 mm or more.
(9) In the SiC ingot according to any one of the above aspects (1) to (8), a diameter may be 145 mm or more.
(10) In the SiC ingot according to any one of the above aspects (1) to (8), a diameter may be 195 mm or more.
(11) A method for manufacturing a SiC substrate according to a second aspect includes: a step of producing the SiC ingot according to any one of the above aspects (1) to (10); and a step of slicing the SiC ingot.
(12) A method for manufacturing a SiC substrate according to a third aspect includes: a step of preparing the SiC ingot according to any one of the above aspects (1) to (10); and a step of slicing the SiC ingot.

The SiC ingot according to the above aspect has excellent crystal quality and processing performance. In addition, the method for manufacturing a SiC substrate according to the above aspect has excellent production efficiency.

### BRIEF DESCRIPTION OF THE DRAWINGS

FIG. 1 is a cross-sectional view of a SiC ingot according to the present embodiment.
FIG. 2 is a plan view of the SiC ingot according to the present embodiment.
FIG. 3 is a diagram for explaining a method for manufacturing a SiC ingot according to the present embodiment.
FIG. 4 is a diagram for explaining a method for manufacturing a SiC ingot according to the present embodiment.

### DETAILED DESCRIPTION OF THE INVENTION

Hereinafter, a SiC ingot and the like according to the present embodiment will be described in detail with reference to the drawings as appropriate. In the drawings which will be used in the following description, featured portions may be enlarged for convenience in order to make the features of the present embodiment easy to understand, and the dimensional ratios of constituent elements may be different from the actual ones. The materials, dimensions, and the like which will be exemplified in the following description are examples, and the present invention is not limited thereto and can be carried out with appropriate modifications without changing the features of the present invention.

In the present specification, an individual orientation is indicated by [ ], a collective orientation is indicated by < >, an individual plane is indicated by ( ), and a collective plane is indicated by { }. For negative indices, a "-" (bar) is customarily placed above the number in crystallography, but in the present specification, a negative sign is placed before the number.

First, directions are defined as follows. A crystal growth direction of a SiC ingot 10 is defined as a Z direction. The Z direction is a height direction of the SiC ingot 10 in a cylindrical shape. One direction in a plane orthogonal to the Z direction is defined as an X direction. The X direction is, for example, a <11-20> direction. For example, a +X direction is defined as a [11-20] direction, and a -X direction is defined as a [-1-120] direction. In addition, in a plane orthogonal to the Z direction, a direction orthogonal to the X direction is defined as a Y direction. The Y direction is, for example, a <1-100> direction.

### "SiC ingot"

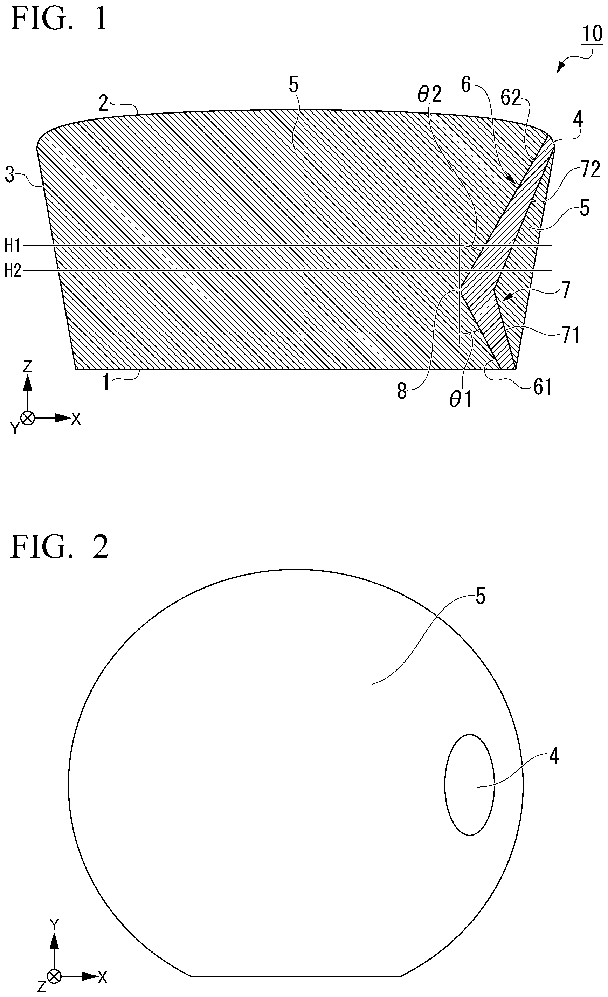

FIG. 1 is a cross-sectional view of the SiC ingot 10 according to the present embodiment. FIG. 2 is a plan view of the SiC ingot 10 according to the present embodiment in the Z direction.

The SiC ingot 10 is a columnar body including a seed crystal and a crystal growth portion obtained by performing crystal growth on the seed crystal. The SiC ingot 10 may be one that has been processed into a cylindrical shape or one that has not yet been processed into a cylindrical shape. The SiC ingot 10 is a single crystal of SiC obtained by performing the crystal growth from a first end 1 toward a second end 2. The first end 1 and the second end 2 are connected to each other by a side wall 3. The SiC ingot 10 may expand in diameter from the first end 1 toward the second end 2, or may be cylindrical with a constant diameter. The first end 1 is, for example, a (0001) plane (a Si plane) or a plane inclined by an offset angle from the (0001) plane. The second end 2 is, for example, a (000-1) plane (a C plane) or a plane inclined by an offset angle from the (000-1) plane. The second end 2 faces the first end 1. For example, the first end 1 and the second end 2 may have an offset angle of 0.1 degrees or more and 8 degrees or less in the <11-20> direction, and may have no offset angle in the <1-100> direction. The offset angle of the SiC ingot 10 is not limited to this example.

The diameter D of the SiC ingot 10 is, for example, 145 mm or more, preferably 149 mm or more, more preferably 155 mm or less, and even more preferably 151 mm or less. The diameter D of the SiC ingot 10 is, for example, 195 mm or more, preferably 199 mm or more, more preferably 205 mm or less, and further preferably 201 mm or less. The diameter D of the SiC ingot 10 is, for example, 305 mm or less.

The diameter D of the SiC ingot 10 is the minimum diameter of the SiC ingot 10 and corresponds to the minimum value of the diameters of the SiC substrates that can be acquired. For example, in a case in which the SiC ingot 10 expands in diameter from the first end 1 toward the second end 2, the diameter of the first end 1 corresponds to the diameter D of the SiC ingot 10. For example, in a case in which the SiC ingot 10 is cylindrical with a constant diameter, the diameter of any cross section obtained by cutting the SiC ingot 10 along a surface orthogonal to the Z direction corresponds to the diameter D of the SiC ingot 10. The SiC ingot 10 may be, for example, one from which a 6-inch substrate can be acquired, or one from which an 8-inch substrate can be acquired.

The thickness (ingot length) of the SiC ingot 10 is, for example, 10 mm or more, preferably 20 mm or more, more preferably 30 mm or more, even more preferably 40 mm or more, and particularly preferably 50 mm or more. The thickness of the SiC ingot 10 is preferably, for example, 300 mm or less. The thicker the SiC ingot 10, the more SiC substrates can be acquired. The thickness of the SiC ingot 10 may hereinafter be referred to as an ingot length.

The SiC ingot 10 includes a facet 4 and a step-flow growth region 5.

The SiC ingot 10 is obtained by performing the crystal growth on a SiC seed crystal. In order to suppress the occurrence of heterogeneous polymorphs, a seed crystal having an offset angle relative to the {0001} plane is often used. The offset angle is, for example, 3.5° or more and 4.5° or less, and is preferably 4°. By performing step-flow growth of SiC on the seed crystal, the occurrence of heterogeneous polymorphs can be suppressed. Even in a case in which the SiC ingot 10 is obtained through the step-flow growth, a part of a crystal growth surface becomes parallel to the (0001) plane, and a surface parallel to the (0001) plane is exposed on the crystal growth surface. On the surface parallel to the (0001) plane, crystals grow perpendicular to the (0001) plane, and thus the step-flow growth is not performed. The facet 4 is a region in which the crystals have grown perpendicular to the (0001) plane. The step-flow growth region 5 is a region in which the crystals have grown through the step-flow growth. The step-flow growth region 5 has an offset angle of, for example, 3.5° or more and 4.5° or less with respect to the {0001 } plane.

In a plan view in the Z direction, the facet 4 and the step-flow growth region 5 have different colors, and a boundary between them can be visually observed. This is because the facet 4 and the step-flow growth region 5 have different patterns in crystal growth. The facet 4 is visually observed as a region darker in color than the step-flow growth region 5.

The boundary between the facet 4 and the step-flow growth region 5 can be determined visually, but may also be determined by the following procedure. First, an image of a cut surface to be measured is acquired. For example, the image is acquired by polishing both surfaces of the substrate and using a scanner. As the scanner, for example, a flatbed scanner manufactured by Canon Inc. can be used. Next, the acquired image is converted into an HLS color space consisting of hue, luminance, and saturation, and the luminance is calculated. Then, in the image converted into a luminance distribution, a circle of pixels with a radius X is drawn with an arbitrary pixel as the center of the circle. In a case in which there are pixels within this circle with a luminance difference from the central pixel of Y or more, the pixel at the center of the circle becomes a pixel that is a facet candidate. In a case in which there are no pixels within this circle with a luminance difference from the central pixel of Y or more, the pixel at the center of the circle becomes a pixel that is not a facet candidate. A similar treatment is then performed on all pixels of the image, and the pixels are classified as either pixels that are facet candidates or pixels that are not facet candidates. Then, the boundary between the pixels that are facet candidates and the pixels that are not facet candidates is detected, and the inside of this region can be considered as a facet. Among the pixels that are facet candidates, pixels that are isolated from other pixels that are facet candidates can be determined not to be facet candidates. The radius X of the circle and the luminance difference Y are set according to the size and number of pixels of the image. These settings involve setting values that do not cause a large discrepancy between the visual observation result and the determination result. For example, in a case in which a 640 pixel × 480 pixel image including a 150 mm-diameter wafer is used, the radius X is set to 9 pixels and the luminance difference Y is set to 4 W·sr⁻¹·m⁻².

Even in an XZ cut surface, the boundary between the facet 4 and the step-flow growth region 5 can be visually determined. Hereinafter, in the boundary between the facet 4 and the step-flow growth region 5, a boundary located on a side of the center of the SiC ingot 10 is referred to as an inner boundary 6, and a boundary located on an outer side from the inner boundary is referred to as an outer boundary 7.

The inner boundary 6 includes a first inclined surface 61 and a second inclined surface 62. The first inclined surface 61 is an inclined surface that is inclined in the -X direction with respect to the Z direction. The second inclined surface 62 is an inclined surface that is inclined in the +X direction with respect to the Z direction. The first inclined surface 61 is located, for example, closer to the first end 1 than the second inclined surface 62.

Similarly, the outer boundary 7 includes a first inclined surface 71 and a second inclined surface 72. The first inclined surface 71 is an inclined surface that is inclined in the -X direction with respect to the Z direction. The second inclined surface 72 is an inclined surface that is inclined in the +X direction with respect to the Z direction. The first inclined surface 71 is located, for example, closer to the first end 1 than the second inclined surface 72.

At the boundary between the first inclined surface 61 and the second inclined surface 62, there is an inflection point 8 at which an inclination angle with respect to the Z direction changes discontinuously. For example, it is preferable that the inflection point 8 be located on a side of the first end 1 from a plane H1 that passes through the center of the SiC ingot 10 in the Z direction (the center position of the ingot length) and is parallel to the XY plane.

In addition, it is more preferable that the inflection point 8 be located on a side of the first end 1 from a plane H2 that passes through a position shifted by 40% of the ingot length in the Z direction from the first end 1 and is parallel to the XY plane. In addition, it is more preferable that the inflection point 8 be located on a side of the first end 1 from a plane that passes through a position shifted by 30% of the ingot length in the Z direction from the first end 1 and is parallel to the XY plane, it is more preferable that the inflection point 8 be located on a side of the first end 1 from a plane that passes through a position shifted by 20% of the ingot length in the Z direction from the first end 1 and is parallel to the XY plane, and it is more preferable that the inflection point 8 be located on a side of the first end 1 from a plane that passes through a position shifted by 10% of the ingot length in the Z direction from the first end 1 and is parallel to the XY plane.

The further the inflection point 8 is away from the first end 1, the more the facet 4 moves to an offset downstream side (-X direction: [-1-120] direction). That is, as the inflection point 8 is further away from the first end 1, the facet 4 is positioned on the significantly more inward side of the SiC ingot 10. As the facet 4 is positioned on the significantly more inward side of the SiC ingot 10, the plane coordinates of the facet 4 when viewed in the Z direction at the height position of the inflection point 8 and the plane coordinates of the facet 4 checked at the second end 2 are likely to deviate significantly from each other.

In the actual SiC ingot 10, only the plane coordinates of the facet 4 at the first end 1 or the second end 2 can be checked, and the plane coordinates of the facet 4 inside can only be estimated. The facet 4 has a lower resistance value than the step-flow growth region 5, and in the facet 4, the light absorption coefficient also changes. For this reason, in a case in which laser processing is performed, it is necessary to adjust conditions such as a laser output and a cutting speed. For example, if the facet 4 is present at a position which is estimated from the plane coordinates of the facet 4 at the second end 2 that there will be no facet 4 even in the inside, the laser output may be insufficient during the laser processing. As described above, as the facet 4 is positioned on the significantly more inward side of the inner side of the SiC ingot 10, the height position of the inflection point 8 and the planar position of the facet 4 at the second end 2 are likely to deviate significantly from each other, and thus the risk of errors occurring during processing is increased.

For this reason, when the inflection point 8 is located on a side of the first end 1, the processing stability of the SiC ingot 10 is improved. In addition, the closer the inflection point 8 is located to a side of the first end 1, the more the processing stability of the SiC ingot 10 is improved.

The facet 4 is preferably located on the offset upstream side of the SiC ingot 10. That is, the facet 4 is preferably located in the +X direction (the [11-20] direction) from a plane (a YZ plane) that passes through the center in the X direction and is orthogonal to the X direction. As described above, the facet 4 is not positioned on the too inward side of the SiC ingot 10, and thus the processing stability of SiC ingot 10 is improved.

The first inclined surface 61 is inclined, for example, in the -X direction at an inclination angle θ1 with respect to the Z direction. The second inclined surface 62 is inclined, for example, in the +X direction at an inclination angle θ2 with respect to the Z direction. The inclination angle θ1 is an absolute value of an interior angle between the first inclined surface 61 and the Z direction. The inclination angle θ2 is an absolute value of an interior angle between the second inclined surface 62 and the Z direction. The inclination angle θ1 is preferably smaller than the inclination angle θ2. By making the inclination angle θ1 smaller than the inclination angle θ2, it is possible to prevent the facet 4 from being positioned on the significantly more inward side of the SiC ingot 10. In addition, when the inclination angle θ2 is greater than the inclination angle θ1, the plane coordinates of the facet 4 are significantly shifted to the outer side of the SiC ingot 10 at the height position subsequent to the inflection point 8. The outer side of the SiC ingot 10 is often outside the region at which the SiC substrate is acquired, and by making the inclination angle θ2 larger than the inclination angle θ1, the facet 4 can be excluded from the region at which the SiC substrate is acquired.

FIG. 1 illustrates a case in which the inclination angle θ1 of the first inclined surface 61 and the inclination angle θ2 of the second inclined surface 62 are constant, but the inclination angle θ1 of the first inclined surface 61 and the inclination angle θ2 of the second inclined surface 62 may change depending on the position in the Z direction. In this case, the average inclination angles of the first inclined surface 61 and the second inclined surface 62 are treated as the inclination angle θ1 and the inclination angle θ2. The average inclination angle is an average value of inclination angles measured at five different positions in the Z direction.

It is preferable that the first inclined surface 61 include a portion at which the inclination angle θ1 is 5° or more, and it is more preferable that the first inclined surface 61 include a portion at which the inclination angle θ1 is 15° or more. Similarly, it is preferable that the second inclined surface 62 include a portion at which the inclination angle θ2 is 5° or more, and it is more preferable that the second inclined surface 62 include a portion at which the inclination angle θ2 is 15° or more. It is more preferable that both the first inclined surface 61 and the second inclined surface 62 include portions at which the inclination angle θ1 and the inclination angle θ2 are 5° or more. In addition, it is more preferable that at least one of the first inclined surface 61 and the second inclined surface 62 include a portion at which the inclination angle θ1 or the inclination angle θ2 is 15° or more.

In addition, it is preferable that the area of the facet 4 at the first end 1 be larger than the area of the facet 4 at the second end 2. The first end 1 corresponds to a portion obtained by performing the crystal growth at the initial stage of the crystal growth of the SiC ingot 10 in comparison with the second end 2. In the initial stage of the crystal growth of the SiC single crystal, the crystal growth is not stable. By providing the facet 4 with a sufficient area on a side of the first end 1, the occurrence of heterogeneous polymorphs can be further suppressed. In addition, if the area of the facet 4 at the second end 2 that corresponds to the later stage of the crystal growth is small, it is easy to process the SiC ingot 10.

### "Method for manufacturing SiC ingot"

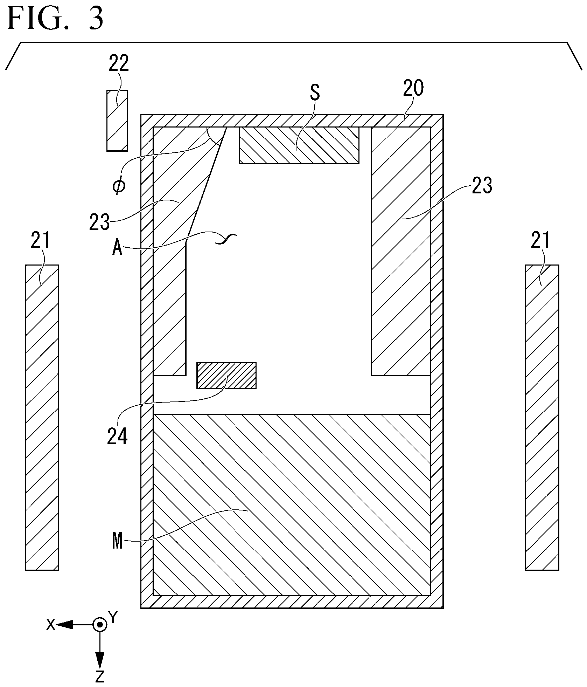

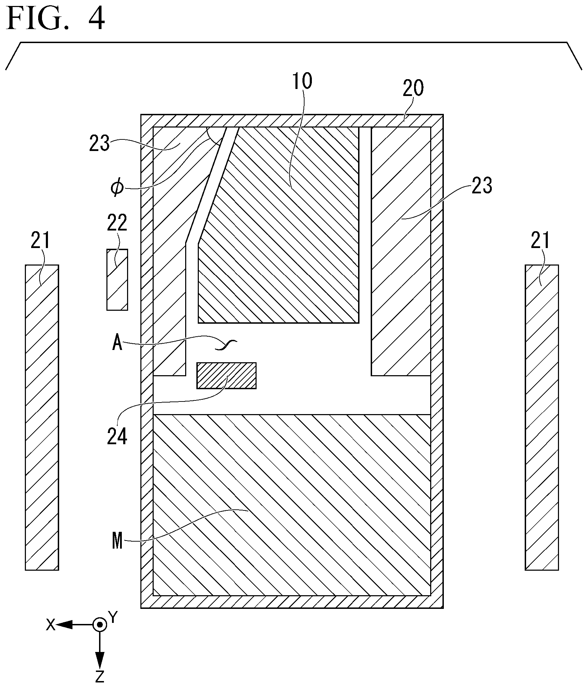

Next, a method for manufacturing the SiC ingot 10 according to the present embodiment will be described. FIGS. 3 and 4 are diagrams for explaining the method for manufacturing the SiC ingot 10 according to the present embodiment.

A apparatus for manufacturing the SiC ingot 10 includes a crucible 20, a heating coil 21, a heat insulating member 22, a guide member 23, and a shielding member 24.

The crucible 20 is made of, for example, graphite. The crucible 20 encloses a growth space A. A seed crystal S and a SiC raw material M are disposed in the growth space A of the crucible 20. The heating coil 21 surrounds the outer periphery of the crucible 20. The heating coil 21 heats the SiC raw material M. The heated SiC raw material M sublimes. Gas sublimated from the SiC raw material M is recrystallized on the surface of the seed crystal S, and thus the crystal growth of the SiC ingot 10 is caused.

The atmospheric pressure in the growth space A during the crystal growth is more than 0.3 Torr and less than 50 Torr. The atmospheric pressure in the growth space A during the crystal growth is, for example, 10 Torr. If the pressure in the growth space A is too low, a dopant element cannot be sufficiently incorporated into the crystal, and the resistivity of the SiC ingot 10 becomes high. The resistivity of the SiC ingot 10 is a parameter that affects the electrical discharge machining. In addition, if the pressure in the growth space A is too high, a sufficient amount of a sublimation gas is not generated from the SiC source material M; and as a result, productivity decreases.

The growth space A is, for example, in an argon and nitrogen gas atmosphere. The crucible 20 is heated while being rotated at a constant speed. The rotation speed of the crucible is, for example, 5 rpm. The temperature of the crucible 20 is, for example, 2300°C at the bottom portion and 2000°C at the top portion.

The heat insulating member 22 is movable in the Z direction. The position of the heat insulating member 22 in the Z direction changes as the crystal growth of the SiC ingot 10 is performed. The growth process of the SiC ingot 10 is divided into two processes of a first growth process and a second growth process, and the position of the insulation member 22 in the Z direction is adjusted in the two processes. The first growth process includes the beginning of growth and the second growth process includes the end of growth. The position of the heat insulating member 22 in the Z direction is set to 5 mm above the center position of the crystal growth surface for the SiC ingot 10 during the first growth process and is set to 5 mm below the center position of the crystal growth surface for the SiC ingot 10 during the second growth process. The boundary point between the first growth process and second growth process is in a range of 10% to 55% of the final growth amount. For example, in a case where the boundary point is 30% of the final growth amount, the first growth process is a process in which the growth of the SiC ingot 10 is progressed to 30% of the final growth amount, and the second growth process is a process in which the growth of the SiC ingot 10 is progressed from 30% of the final growth amount to the end. In addition, "5 mm above the center position of the crystal growth surface" means "a position that is 5 mm close to the seed crystal S from the center position of the crystal growth surface in the Z direction", and "5 mm below the center position of the crystal growth surface" means "a position that is 5 mm away from the seed crystal S from the center position of the crystal growth surface in the Z direction".

The guide member 23 is made of, for example, graphite or high melting point ceramic. For example, the shape of the guide member 23 on the offset upstream side (+X direction) is different from that on the offset downstream side (-X direction). The inner side surface of the guide member 23 located on the offset upstream side has an inclination angle φ with respect to the horizontal direction (direction perpendicular to the Z direction) which varies depending on the position in the Z direction. The inclination angle φ is 50° or more and 85° or less in a range of the position in the Z direction of the crystal growth surface in the first growth process, and the inclination angle φ is 80° or more and 90° or less in a range of the position in the Z direction of the crystal growth surface in the second growth process. For example, in a case where the boundary point between the first growth process and second growth process is 30% of the final growth amount, the inclination angle φ is 50° or more and 85° or less at a position to 30% of the final growth amount of the SiC ingot 10 and the inclination angle φ is 80° or more and 90° or less at a position from 30% to 100% of the final growth amount of the SiC ingot 10. In addition, the inner side surface of the guide member 23 located on the offset downstream side has an inclination angle with respect to the horizontal direction (direction perpendicular to the Z direction) which is 80° or more and 90° or less regardless of the position in the Z direction.

The shielding member 24 is located between either one of the seed crystal S or the SiC ingot 10 during crystal growth and the SiC raw material M. The shielding member 24 is disposed at a position at which a distance from the surface of the SiC ingot 10 after crystal growth is 20 mm or more. The shielding member 24 may have any shape and has a thickness of, for example, 5 mm or more. The shielding member 24 covers only the offset upstream side of the SiC ingot 10 when viewed in the Z direction. For example, the shielding member 24 covers only the offset upstream side of the SiC ingot 10 in a sector shape when viewed in the Z direction.

The heat insulating member 22, the guide member 23, and the shielding member 24 are disposed at the positions described above, and then the crystal growth of the SiC ingot 10 is performed on the seed crystal S. By arranging the heat insulating member 22, the guide member 23, and the shielding member 24 at predetermined positions, the temperature distribution within the growth space A can be controlled, and the SiC ingot 10 having the desired facet 4 can be produced.

In addition, the produced SiC ingot 10 can be processed into a cylindrical shape and sliced to produce a SiC substrate.

In the SiC ingot 10 according to the present embodiment, a shape of the facet 4 is controlled, and thus the SiC ingot 10 has high crystal quality and it is easy to process the SiC ingot 10. In the SiC ingot 10 according to the present embodiment, in the initial stage of the crystal growth, the facet 4 moves to the offset downstream side, and the first inclined surface 61 is formed. In the initial stage of the crystal growth when the crystal growth is not stable, the occurrence of heterogeneous polymorphs in the SiC ingot 10 is suppressed by growing the facet 4 toward the offset downstream side. In addition, in the SiC ingot 10 according to the present embodiment, in the later stage of the crystal growth, the facet 4 moves to the offset upstream side, and the second inclined surface 62 is formed. By moving the position of the facet 4 to the outer side of the SiC ingot 10, the proportion of the facet 4 within the SiC substrate acquisition region can be reduced; and thereby, it becomes easier to process the SiC ingot 10.

### "Method for manufacturing SiC substrate"

A method for manufacturing a SiC substrate of a first embodiment includes a step of manufacturing the SiC ingot 10 of the embodiment described above by any of the methods described above, and a step of slicing the SiC ingot 10.

As the step of slicing the SiC ingot, for example, a method in which the SiC ingot is processed with a laser to create cracks and cut out the SiC substrates can be used. The SiC ingot 10 may be processed into a cylindrical shape before the step of slicing the SiC ingot 10.

A method for manufacturing a SiC substrate of a second embodiment includes a step of preparing the SiC ingot 10 of the embodiment described above, and a step of slicing the SiC ingot 10.

The step of preparing the SiC ingot 10 may include obtaining the SiC ingot 10 of the above-mentioned embodiment from other companies, and the SiC ingot 10 may be a boule, provided that the boule meets the features of the SiC ingot 10 of the above-mentioned embodiment.

The step of slicing the SiC ingot 10 is the same as that in the first embodiment. The SiC ingot 10 may be processed into a cylindrical shape before the step of slicing the SiC ingot 10.

As described above, the preferable embodiments of the present invention have been described in detail, the present invention is not limited to specific embodiments, and various modifications and changes can be made within the scope of the features of the present invention described in the claims.

### [Examples]

### "Example 1"

The seed crystal having a diameter of 8 inches (200 mm) was prepared. Then, using the manufacturing apparatus shown in FIGS. 3 and 4, the crystal growth of the SiC ingot was performed on the seed crystal to 30 mm.

The growth space A was in an Ar and N₂ gas atmosphere at a pressure of 10 Torr. The temperature of the crucible 20 was set to 2300°C at the bottom portion and 2000°C at the top portion. The rotation speed of the crucible 20 was set to 5 rpm. The inclination angle φ of the inner side surface of the guide member 23 located on the offset upstream side was set to 70° at a position to 30% of the final growth amount of the SiC ingot (the position in the Z direction of the crystal growth surface in the first growth process described later) and was set to 85° at a position from 30% to 100% of the final growth amount of the SiC ingot (the position in the Z direction of the crystal growth surface in the second growth process described later). In addition, the inclination angle of the inner side surface of the guide member 23 located on the offset downstream side was set to 90° regardless of the position in the Z direction. In addition, the shielding member 24 was disposed to cover only the offset upstream side in a sector shape.

As the crystal growth progressed, the position of the heat insulating member 22 was changed. The process in which the growth of the SiC ingot 10 was progressed to 30% of the final growth amount was defined as the first growth process, and the process in which the growth of the SiC ingot 10 was progressed from 30% of the final growth amount to the end was defined as the second growth process. The position of the heat insulating member 22 in the Z direction was set to 5 mm above the center position of the crystal growth surface for the SiC ingot 10 during the first growth process, and the position of the heat insulating member 22 in the Z direction was set to 5 mm below the center position of the crystal growth surface for the SiC ingot 10 during the second growth process.

Under the above-described conditions, the SiC ingot of Example 1 was produced. Then, the cross section of the produced SiC ingot was checked, and the shape of the inner boundary between the facet and the step-flow growth region was checked. The SiC ingot included a first end that was opposite the growth surface of the seed crystal and a second end that was the growth surface. The inner boundary of the facet included the first inclined surface and the second inclined surface. The first inclined surface was closer to the first end than the second inclined surface. The inclination angle of the first inclined surface with respect to the crystal growth direction was smaller than the inclination angle of the second inclined surface with respect to the crystal growth direction. The first inclined surface included a portion in which an absolute value of an inclination angle with respect to the crystal growth direction was 5° or more. The second inclined surface included a portion in which an absolute value of an inclination angle with respect to the crystal growth direction was 15° or more. The inflection point between the first inclined surface and the second inclined surface was located at a position shifted by 20% of the ingot length in the Z direction from the first end. The facet was located in a [11-20] direction from a plane that passed through a center in a <11-20> direction and was orthogonal to the <11-20> direction.

In addition, under the same conditions, 20 SiC ingots of Example 1 were produced, and the SiC ingots were evaluated. Specifically, the quality yield of the SiC ingots and the average time required to process the SiC ingots were measured.

The quality yield of the SiC ingots was evaluated by evaluating the presence or absence of heterogeneous polymorphs. Specifically, the SiC ingot was cut with a wire saw at positions shifted 2 mm from each of the first end and the second end of the SiC ingot to acquire substrates for evaluation. The thickness of the substrate was 0.5 mm. Both surfaces of this substrate were polished, and then an X-ray topography (XRT) (diffraction vector g: 1-100) transmission image was acquired to check the presence or absence of heterogeneous polymorphs and micropipes resulting therefrom.

The time required to acquire a SiC wafer from a SiC ingot processed into a cylindrical shape was measured as the time required to process the SiC ingot. Laser processing was used to acquire the SiC wafer from the SiC ingot. The scan pitch of the laser was set to 200 µm, the feed speed of the laser scan was set to 200 mm/sec, the number of scans was set to 1, the acceleration/deceleration time was set to 0.1 sec, and the inter-line movement time was set to 0.1 sec. The acceleration/deceleration time was the time required to accelerate and decelerate when scanning with the laser in one direction and then scanning with the laser in an opposite direction. The inter-line movement time indicated the time required for movement in a second direction in a treatment in which laser scanning in a first direction and the movement in the second direction orthogonal to the first direction were repeated.

In the SiC ingots of Example 1, 19 SiC ingots among 20 SiC ingots did not include the heterogeneous polymorphs, and the quality yield was 95%. In addition, the time required to slice the SiC ingots of Example 1 into wafers was 18.2 minutes per wafer.

### "Example 2"

The seed crystal having a diameter of 8 inches (200 mm) was prepared. Then, using the manufacturing apparatus shown in FIGS. 3 and 4, the crystal growth of the SiC ingot was performed on the seed crystal to 30 mm.

The process in which the growth of the SiC ingot was progressed to 50% of the final growth amount was defined as the first growth process, and the process in which the growth of the SiC ingot was progressed from 50% of the final growth amount to the end was defined as the second growth process. The position of the heat insulating member 22 in the Z direction was set to 5 mm above the center position of the crystal growth surface for the SiC ingot during the first growth process, and the position of the heat insulating member 22 in the Z direction was set to 5 mm below the center position of the crystal growth surface for the SiC ingot during the second growth process. The inclination angle φ of the inner side surface of the guide member 23 located on the offset upstream side was set to 70° at a position to 50% of the final growth amount of the SiC ingot (the position in the Z direction of the crystal growth surface in the first growth process) and was set to 85° at a position from 50% to 100% of the final growth amount of the SiC ingot (the position in the Z direction of the crystal growth surface in the second growth process). Other conditions were the same as those in Example 1 and the SiC ingot of Example 2 was produced.

The cross section of the produced SiC ingot was checked, and the shape of the inner boundary between the facet and the step-flow growth region was checked. The SiC ingot included a first end that was opposite the growth surface of the seed crystal and a second end that was the growth surface. The inner boundary of the facet included the first inclined surface and the second inclined surface. The first inclined surface was closer to the first end than the second inclined surface. The inclination angle of the first inclined surface with respect to the crystal growth direction was smaller than the inclination angle of the second inclined surface with respect to the crystal growth direction. The first inclined surface included a portion in which an absolute value of an inclination angle with respect to the crystal growth direction was 5° or more. The second inclined surface included a portion in which an absolute value of an inclination angle with respect to the crystal growth direction was 15° or more. The inflection point between the first inclined surface and the second inclined surface was located at a position shifted by 40% of the ingot length in the Z direction from the first end. The facet was located in a [11-20] direction from a plane that passed through a center in a <11-20> direction and was orthogonal to the <11-20> direction.

In addition, under the same conditions, 20 SiC ingots of Example 2 were produced, and the SiC ingots were evaluated. In the SiC ingots of Example 2, 19 SiC ingots among 20 SiC ingots did not include the heterogeneous polymorphs, and the quality yield was 95%.

The time required to slice the SiC ingots into wafers was measured under the same conditions as Example 1. The time required to slice the SiC ingots of Example 2 into wafers was 18.6 minutes per wafer.

### "Comparative Example 1"

Comparative Example 1 differs from Example 1 in that a SiC ingot was produced using an apparatus for manufacturing a SiC ingot that did not have the guide member 23 and the shielding member 24. In addition, in the process of manufacturing the SiC ingot of Comparative Example 1, the position of the heat insulating member 22 in the Z direction was always 5 mm above the center position of the crystal growth surface for the SiC ingot from the initial stage to the end of the growth.

Under the above-described conditions, the SiC ingot of Comparative Example 1 was produced. Then, the cross section of the produced SiC ingot was checked, and the shape of the inner boundary between the facet and the step-flow growth region was checked. The inner boundary consisted of the first inclined surface inclined in the [-1-120] direction with respect to the crystal growth direction.

In addition, under the same conditions, 20 SiC ingots of Comparative Example 1 were produced, and the quality yield of the SiC ingots and the average time required to process the SiC ingots were measured.

The quality yield of the SiC ingots in Comparative Example 1 was 95%. In addition, the time required to slice the SiC ingots of Comparative Example 1 into wafers was 20.4 minutes per wafer.

### "Comparative Example 2"

Comparative Example 2 differs from Example 1 in that a SiC ingot was produced using an apparatus for manufacturing a SiC ingot that did not have the guide member 23 and the shielding member 24. In addition, in the process of manufacturing the SiC ingot of Comparative Example 2, the position of the heat insulating member 22 in the Z direction was always 5 mm below the center position of the crystal growth surface for the SiC ingot from the initial stage to the end of the growth.

Under the above-described conditions, the SiC ingot of Comparative Example 2 was produced. Then, the cross section of the produced SiC ingot was checked, and the shape of the inner boundary between the facet and the step-flow growth region was checked. The inner boundary consisted of the second inclined surface inclined in the [11-20] direction with respect to the crystal growth direction.

In addition, under the same conditions, 20 SiC ingots of Comparative Example 2 were produced, and the quality yield of the SiC ingots and the average time required to process the SiC ingots were measured.

The quality yield of the SiC ingots in Comparative Example 2 was 70%. In addition, the time required to slice the SiC ingots of Comparative Example 2 into wafers was 17.8 minutes per wafer.

### "Comparative Example 3"

In Comparative Example 3, the position of the heat insulating member 22 during the crystal growth was changed from that in Example 1. In Comparative Example 3, the process in which the growth of the SiC ingot was progressed to 60% of the final growth amount was defined as the first growth process, and the process in which the growth of the SiC ingot was progressed from 60% of the final growth amount to the end was defined as the second growth process. The position of the heat insulating member 22 in the Z direction was 5 mm above the center position of the crystal growth surface for the SiC ingot during the first growth process, and the position of the heat insulating member 22 in the Z direction was 5 mm below the center position of the crystal growth surface for the SiC ingot during the second growth process. The inclination angle φ of the inner side surface of the guide member 23 located on the offset upstream side was set to 70° at a position to 70% of the final growth amount of the SiC ingot and was set to 85° at a position from 70% to 100% of the final growth amount of the SiC ingot.

Under the above-described conditions, the SiC ingot of Comparative Example 3 was produced. Then, the cross section of the produced SiC ingot was checked, and the shape of the inner boundary between the facet and the step-flow growth region was checked. The inner boundary included the first inclined surface and the second inclined surface. The inflection point between the first inclined surface and the second inclined surface was located at a position shifted by 80% of the ingot length in the Z direction from the first end.

In addition, under the same conditions, 20 SiC ingots of Comparative Example 3 were produced, and the quality yield of the SiC ingots and the average time required to process the SiC ingots were measured.

The quality yield of the SiC ingots in Comparative Example 3 was 95%. In addition, the time required to slice the SiC ingots of Comparative Example 3 into wafers was 19.5 minutes per wafer.

### "Comparative Example 4"

The seed crystal having a diameter of 8 inches (200 mm) was prepared. Then, using the manufacturing apparatus shown in FIGS. 3 and 4, the crystal growth of the SiC ingot was performed on the seed crystal to 30 mm.

The process in which the growth of the SiC ingot was progressed to 70% of the final growth amount was defined as the first growth process, and the process in which the growth of the SiC ingot was progressed from 70% of the final growth amount to the end was defined as the second growth process. The position of the heat insulating member 22 in the Z direction was set to 5 mm above the center position of the crystal growth surface for the SiC ingot during the first growth process, and the position of the heat insulating member 22 in the Z direction was set to 5 mm below the center position of the crystal growth surface for the SiC ingot during the second growth process. The inclination angle φ of the inner side surface of the guide member 23 located on the offset upstream side was set to 70° at a position to 70% of the final growth amount of the SiC ingot (the position in the Z direction of the crystal growth surface in the first growth process) and was set to 85° at a position from 70% to 100% of the final growth amount of the SiC ingot (the position in the Z direction of the crystal growth surface in the second growth process). Other conditions were the same as those in Example 1 and the SiC ingot of Comparative Example 4 was produced.

The cross section of the produced SiC ingot was checked, and the shape of the inner boundary between the facet and the step-flow growth region was checked. The SiC ingot included a first end that was opposite the growth surface of the seed crystal and a second end that was the growth surface. The inner boundary of the facet included the first inclined surface and the second inclined surface. The first inclined surface was closer to the first end than the second inclined surface. However, the inclination angle of the first inclined surface with respect to the crystal growth direction was larger than the inclination angle of the second inclined surface with respect to the crystal growth direction. The first inclined surface included a portion in which an absolute value of an inclination angle with respect to the crystal growth direction was 5° or more. However, the second inclined surface did not include a portion in which an absolute value of an inclination angle with respect to the crystal growth direction was 15° or more. The inflection point between the first inclined surface and the second inclined surface was located at a position shifted by 60% of the ingot length in the Z direction from the first end. The facet was located in a [11-20] direction from a plane that passed through a center in a <11-20> direction and was orthogonal to the <11-20> direction.

In addition, under the same conditions, 20 SiC ingots of Comparative Example 4 were produced, and the SiC ingots were evaluated. In the SiC ingots of Comparative Example 4, 19 SiC ingots among 20 SiC ingots did not include the heterogeneous polymorphs, and the quality yield was 95%.

The time required to slice the SiC ingots into wafers was measured under the same conditions as Example 1. The time required to slice the SiC ingots of Comparative Example 4 into wafers was 19.1 minutes per wafer.

**Table 1**

| | Example 1 | Example 2 | Comparative Example 1 | Comparative Example 2 | Comparative Example 3 | Comparative Example 4 |
|---|---|---|---|---|---|---|
| Quality yield [%] | 95% | 95% | 95% | 70% | 95% | 95% |
| Processing time [min/wafer] | 18.2 | 18.6 | 20.4 | 17.8 | 19.5 | 19.1 |

### EXPLANATION OF REFERENCES

1 First end
2 Second end
3 Side wall
4 Facet
5 Step-flow growth region
6 Inner boundary
7 Outer boundary
8 Inflection point
10 SiC ingot
20 Crucible
21 Heating coil
22 Heat insulating member
23 Guide member
24 Shielding member
61, 71 First inclined surface
62, 72 Second inclined surface
A Growth space
H1, H2 Plane
M SiC raw material
S Seed crystal
θ1, θ2, φ Inclination angle

## Claims

1. A SiC ingot comprising a step-flow growth region and a facet,
wherein, in a cut surface that passes through a center and is in a <11-20> direction, an inner boundary between the facet and the step-flow growth region has a first inclined surface inclined in a [-1-120] direction with respect to a crystal growth direction and a second inclined surface inclined in a [11-20] direction with respect to the crystal growth direction, and
wherein an inflection point between the first inclined surface and the second inclined surface is located on a side of a first end, which is a Si plane or a plane inclined by an offset angle from the Si plane, from a center position of an ingot length.

2. The SiC ingot according to claim 1, wherein the inflection point between the first inclined surface and the second inclined surface is located on a side of the first end side from a position shifted by 40% of the ingot length in the crystal growth direction from the first end.

3. The SiC ingot according to claim 1 or 2, wherein the first inclined surface is closer to the first end than the second inclined surface.

4. The SiC ingot according to any one of claims 1 to 3, wherein an inclination angle of the first inclined surface with respect to the crystal growth direction is smaller than an inclination angle of the second inclined surface with respect to the crystal growth direction.

5. The SiC ingot according to any one of claims 1 to 4, wherein the first inclined surface and the second inclined surface include a portion in which an absolute value of an inclination angle with respect to the crystal growth direction is 5° or more.

6. The SiC ingot according to any one of claims 1 to 5, wherein at least one of the first inclined surface and the second inclined surface includes a portion in which an absolute value of an inclination angle with respect to the crystal growth direction is 15° or more.

7. The SiC ingot according to any one of claims 1 to 6, wherein the facet is located in a [11-20] direction from a plane that passes through a center in a <11-20> direction and is orthogonal to the <11-20> direction.

8. The SiC ingot according to any one of claims 1 to 7, wherein the ingot length is 10 mm or more.

9. The SiC ingot according to any one of claims 1 to 8, wherein a diameter is 145 mm or more.

10. The SiC ingot according to any one of claims 1 to 8, wherein a diameter is 195 mm or more.

11. A method for manufacturing a SiC substrate comprising:
a step of producing the SiC ingot according to any one of claims 1 to 10; and
a step of slicing the SiC ingot.

12. A method for manufacturing a SiC substrate comprising:
a step of preparing the SiC ingot according to any one of claims 1 to 10; and
a step of slicing the SiC ingot.
